# EUROPEAN PATENT SPECIFICATION

(11) **EP 0 238 515 B1**
(45) Date of publication and mention of the grant of the patent: **24.03.1993**
(21) Application number: 86905176.3
(22) Date of filing: 21.08.1986
(51) Int. Cl.: A61M 16/00

(54) **TRACHEOSTOMY CANNULA**
SONDE FÜR LUFTRÖHRENSCHNITTE
CANULE DE TRACHEOTOMIE

(30) Priority: 09.09.1985 BR 8504438
(43) Date of publication of application: 30.09.1987
(73) Proprietor: OLIVEIRA FAUZA, Dario de, 04645-Sâo Paulo, SP (BR)
(72) Inventor: OLIVEIRA FAUZA, Dario de, 04645-Sâo Paulo, SP (BR)
(74) Representative: Hirsch, Marc-Roger
(86) International application number: PCT/BR86/00016
(87) International publication number: WO 87/01293

(56) References cited:
- DE-A- 2 425 476
- US-A- 3 516 407
- US-A- 3 889 688
- US-A- 4 141 364
- US-A- 4 449 523
- US-A- 4 459 984
- US-A- 4 538 606

## Description

The present invention relates to a tracheostomy cannula for introduction into the trachea of a patient to form an artificial airway through the patient's throat, comprising:
- a hollow tube having proximal and distal end portions and a bend intermediate of said end portions so as to form first and second portions of said tube respectively including said proximal and distal end portions of said tube;
- an inflatable cuff consisting in a small balloon;
- conduits for inflating and deflating said cuff.

The present invention refers to a tracheostomy cannula with format and distribution of its components designed to prevent the so-called tracheo-innominate fistula, the smallest stimulus to the coughing reflex and to allow it to be easily cleaned.

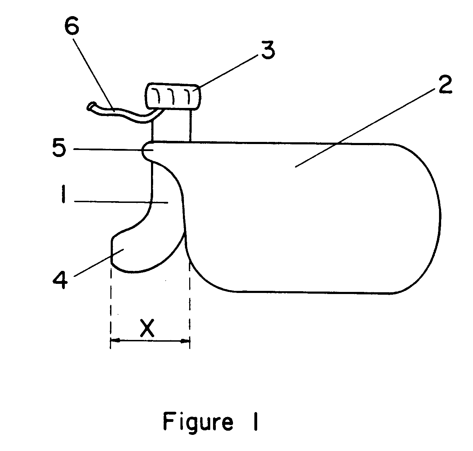

The immense majority of cannulas for tracheostomy follow a basic concept that, in itself, is the model most used today. It consists of a curved tube which acts as a passage for air between the patient's trachea and the environment or the artificial respirator. As it is often necessary to use positive inspiratory pressure, by means of the above-mentioned respirators, this tube is enveloped at its caudal end by a small balloon inflatable with air or liquid, which, by adhering completely to the internal lining of the trachea, by means of a cross section of this organ, prevents the air insufflated by the respirator from escaping into the environment and/or into the larynx and pharynx, to be conducted to the caudal section of the trachea, bronchi and pulmonary alveoli. Yet another function of this small balloon or "cuff" is to support the tube itself inside the trachea. The dimensions of the cannula vary a great deal, especially when considering pediatric models. However, for adults, the limits are quite close and we should note that the distance from the cranial edge to its distal (or caudal) extremity in an orthogonal projection, parallel to the axis of the trachea is of 4 to 5.5 cm. in usual models. This distance is analogous to distance "x", presented in Figure 1 of the invention, to be described later.

Among the many complications of a tracheostomy, we should remember, for a better understanding of the development of the present invention, some that depend on aggressions established by the tube itself and/or by the small balloon. Thus, the longer the cannula tube, the greater the stimulus to coughing and the harder to clean both itself and the trachea. However, the greatest aggravating factor of such cannulas are the lesions to the trachea walls and/or to structures contiguous to the trachea, produced by the possible compression exerted on these tissues by the tube, mainly in its caudal extremity and/or by the small balloon, especially if the pressure in its interior is very high. Such lesions range from a simple ischaemia to real perforations, with resulting secondary complications that are essentially obstructive, infectious and/or hemorrhagic. Among the latter can be noted the so-called tracheo-innominate fistula - an abnormal passage between the trachea and the innominate artery through such perforations. This fearful intercurrent process may cause severe hemorrhages which, when they do occur, lead to a case fatality rate of over 92%. Its occurrence is intimately related to the dimensions of the cannula: the longer it is the greater the risk to the patient. This risk is already uncommonly high, since the distance from the upper limits of the second cartilagineous tracheal ring - cranial limit of the great majority of tracheostomies, up to the upper crossing point of the trachea with the innominate artery is, on average for adults, of 3.3 cm, whereas the same distance measured on the cannula to its caudal extremity is between 4 and 5.5 cm approximately, as mentioned above. Another "inconvenience", not properly a complication, in the use of these tracheostomy cannulas is the need for a tape fitted around the neck of the patient and tied to the proximal extremity of the cannula in order to hold and fix it to its place.

In a scientific publication (A Newly Developed Permanent Tracheostomy Tube, D. Adams, Journal of Laryngology and Otology, 1975, No 89, vol. 7, pages 767 to 769) it is described a model which, among other small improvements, does away with the use of tapes around the neck and lends itself to the prevention of this tracheo-innominate fistula. It consists of a simple tube placed between the skin of the neck and the anterior wall of the trachea, fixed there by its proximal and distal edges. As it does not progress caudally along the interior of the trachea, if it does not do away with, it greatly reduces the risk of the onset of this complication. This cannula only holds the tracheal stoma, without, however, allowing the use of positive inspiratory pressure through artificial respirators, which renders its use impossible in various patients to be tracheostomized. Besides, it is difficult to remove them from the tracheal stoma and these, also, may, although on a smaller scale, lead to infectious, obstructive or hemorrhagic complications.

Also it is known from US-A-3,889,688 a tracheostomy cannula which differs from the cannula according to the invention comprising two inflatable balloons and a collar being not movable, not inflatable and not communicating with the "cuff". The cannula according to said USP 3,889,688 fundamentally differs from the cannula of the invention which renders tracheal lesions impossible to incur.

In short, whatever the model to be considered, there is still the risk of aggression on the part of the components of the cannula, and the consequences tend to be more severe the more caudally these components progress through the interior of the trachea.

With the objective of decreasing the aggressive potential of tracheostomy cannulas, in a general way, but particularly concerning the lesser stimulus to the coughing reflex, ease in cleaning both itself and the trachea, and, principally, preventing a tracheo-innominate fistula, the cannula of the present invention was developed, with its small balloon or "cuff" positioned in the region posterior to the curved tube, which, by this positioning, allows a reduction in its length.

In the tracheostomy cannula according to the invention, the proximal end portion has a radius slightly larger than that of said first portion of the tube, the second portion of said tube extending in a first direction within the trachea after insertion of the distal end portion through a tracheal stoma into patient's throat, and the cuff when inflated extends within patient's trachea only in a second direction substantially opposite to said first direction and has an anterior extension for adhering said inflatable cuff to said tube

The tracheostomy cannula which was developed may be better understood by the description of the diagrams enclosed, which represent:
- Figure 1 - Side view in elevation of the tracheostomy cannula.
- Figure 2 - View in perspective of this same cannula.

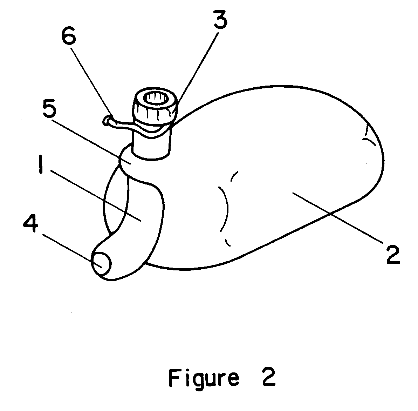

As can be seen by Figures 1 and 2, the tracheostomy cannula, object of this present invention, consists of a curved tube (1) with a circular contour in cross-section, but quite short, with distance "x" equal to about 1 cm which, besides decreasing the coughing reflex and facilitating the cleaning both of itself and of the trachea, drastically reduces or possibly eliminates the risk of occurrence of a tracheo-innominate fistula, since the caudal extremity of the tube, generally, will not reach the region of the crossing of the trachea with the innominate artery, also known as the brachiocephalic trunk. Its extremity, known as proximal (3), in other words, that which is exposed to the environment on introduction of the cannula into the trachea, is equally circular in contour to the cross-section of the tube (1) but with a radius that is slightly larger than that of the tube and adaptable to artificial tracheas. The distal or caudal extremity (4), however, which is inside the trachea, also has a circular contour accompanying the tube; however, its radius is slightly smaller than the radius of the tube, which renders its edge flatter and lessens aggression to the trachea wall since, as is known, it is this edge of the distal extremity that is primarily responsible for lesions.

Its small balloon of "cuff" (2) is placed posteriorly to the tube, with the anterior or caudal portion closely adhering to it. Such positioning of the "cuff" should, also, do away with the risk of the onset of a tracheo-innominate fistula, as a result of a possible agression to the wall of the trachea, since it will be far from the crossing of the trachea with the innominate artery. This "cuff" (2) also has an anterior extension which involves all of the proximal third of the tube (1), to form a "collar" (5) which, together with the configuration of the proximal extremity of the tube (1), will help to fix the cannula to the site, dispensing with the uncomfortable use of tapes around the neck and preventing possible escape of air insufflated under positive pressure into the environment. As the "cuff" (2) can progress as far as the larynx on a level with the vocal cords, it will have great restraining capacity - also, because it will not have a tube crossing it, as in the usual models, consequently with low pressure in its interior, which has been proved, considerably reduces its capacity for aggression to the endotracheal lining. In addition, the contour of this "cuff" (2) in cross-section to the craniocaudal axis, should be similar to the endotracheal lining of the majority of persons on this same plane, or, in other words, elliptical, with a greater latero-lateral axis.

Control of pressure, as also the insufflation and deflation of the "cuff" (2), takes place by means of conduits (6) which may or may not be flexible, and which connect the interior of the "cuff" (2) with the environment, and are fitted with caps, with or without a valve, as in the case of other models.

The material making up the cannula components should be as inert as possible - that is, promote the least possible reaction, or none at all, between the body and the instrument. For instance, polytetrafluorethylene, polyvinyl and many others.

The tracheostomy cannula of the invention may, starting from the basic concept described above, undergo some changes such as in its dimensions, which vary according to the patient's anatomical characteristics; the curved tube may or may not have two "central cavities", or in other words, an internal removable lining, for cases of acute obstruction or to facilitate cleaning; the caudal extremity (4) may not be flat, should this increase the resistance to the air flow in very small cannulas; however, this fact is almost impossible to occur; the proximal extremity may or may not be removable, and, should it be so, will be coupled to the tube, for instance, by means of threading which may or may not lead it to the skin of the patient's neck, should this contribute to fixing the cannula; the tube may have contours other than circular in cross-section; the anterior extension or "collar" (5) may be of varied configurations, ranging from one analogous to that presented in Figures 1 and 2 to that in which it lines all of the intratracheal portion of the tube as far as its distal extremities, which may contribute to increase the restraining capacity of the "cuff" (2) with a resulting low pressure in its interior and/or to better hold the cannula; this same "collar" (5) may or may not be totally or partly adhering to the tube. Its mobility, should it not be adhered, should facilitate sealing of the tracheal stoma, should the dimensions of the trachea, principally the anteroposterior diameter, be extremely variable; the "collar" (5) may or not communicate with the main compartment of the "cuff" (2), just as it may, or not, be of inflatable material; pressure within the "cuff" (2) may or not be controlled by valves, as is already the case in other models; the "cuff" (2) may or may not be fitted with two separate compartments and should it be so, one compartment may be insufflated while the second is not, and vice-versa, alternately, which may decrease aggression to the tracheal wall without, however, loss of function, as occurs in the case of periodic deflation of common "cuffs"; the "cuff" (2) may (preferably) or not have all of its anterior portion adhering to the tube, always, however, retaining some adhesion; contour of the "cuff" may assume forms other than elliptical, according to the anatomical characteristicS of the patient; in some rare cases it may be necessary to wear tapes around the neck, attached to the proximal portion of the cannula; and, finally, the material making up the cannula may be of any kind, preference being given, however, and logically, to the more "inert" types.

The functionS of the tracheostomy cannula, object of this invention, are the same as the "traditional" one. Its insertion into the patient is effected by means of the classic tracheostomy techniques and it is introduced into the trachea with the "cuff" totally deflated.

Once it is in place, it is insufflated. As in existing models, insufflation and deflating, as also the control of pressure inside the "cuff" is done by means of small sealable conduits, flexible or not, that connect the interior of the small balloon to the environment.

## Claims

1. A tracheostomy cannula for introduction into the trachea of a patient to form an artificial airway through the patient's throat, comprising:
- a hollow tube (1) having proximal (3) and distal (4) end portions and a bend intermediate of said end portions so as to form first and second portions of said tube (1) respectively including said proximal (3) and distal (4) end portions of said tube;
- an inflatable cuff (2) consisting in a small balloon;
- an anterior extension for adhering said inflatable cuff (2) to said tube (1)
- conduits (6) for inflating and deflating said cuff (2);
characterized in that
- said proximal end portion (3) has a radius slightly larger than that of said first portion of said tube (1), said second portion of said tube extending in a first direction within the trachea after insertion of said distal end portion (4) through a tracheal stoma into patient's throat, and in that
- said cuff (2) when inflated extends within patient(s) trachea only in a second direction substantially opposite to said first direction and has an anterior extension for adhering said inflatable cuff (2) to said tube (1).

2. A tracheostomy cannula according to claim 1, wherein said inflating and deflating conduits comprises at least one flexible conduit (6).

3. A tracheostomy cannula according to claims 1 or 2, wherein said anterior extension comprises a collar (5).

4. A tracheostomy cannula according to claim 3, wherein said collar (5) is movable along said tube.

5. A tracheostomy cannula according to any one of claims 3 and 4, wherein said collar (5) is inflatable.

6. A tracheostomy cannula according to claim 5, wherein said collar (5) communicates with said cuff (2).

7. A tracheostomy cannula according to any one of claims 1 to 6, wherein said cuff (2) is of such a size when expanded so as to expand within the limits of the tracheal lumen as far as the patient's larynx in order to restrain said tube within the patient's trachea when said tracheostomy cannula is inserted into the trachea of the patient.

8. A tracheostomy cannula according to claim 7, wherein said collar (5) is disposed on said first portion of said tube (1) so as to prevent air leakage through the tracheal stoma when said tracheostomy cannula is inserted into the trachea of the patient.

## Patentansprüche

1. Eine Tracheakanüle zum Einführen in die Trachea eines Patienten um einen künstlichen Durchlass für die Luft durch den Hals des Patienten zu bilden welche aufweist:
- eine hohle Röhre (1) mit proximalen (3) und distalen (4) Endteilen und einem Knie zwischen diesen Endteilen derart, daß in der Röhre eine erste und eine zweite Portion gebildet werden die jeweils die respektiven proximalen (3) und distalen (4) Endteile der Röhre umfassen;
- eine aus einem kleinen Ballon bestehende aufblasbare Muffe (2);
- Leitungen (6) zum Aufblasen und Abblasen dieser Muffe (2);
dadurch gekennzeichnet, daß:
- der genannte proximale Endteil (3) einen etwas größeren Radius wie der des ersten Teils der Röhre (1) aufweist, wobei der zweite Teil sich in eine erste Richtung in der Trachea erstreckt nachdem der genannte distale Endteil (4) durch eine Tracheaöffnung in den Hals des Patienten eingeführt wurde,
und daß
- die Muffe (2), wenn sie aufgeblasen ist, sich im Inneren der Trachea des (der) Patienten lediglich gemäß einer zweiten, der ersten Richtung im wesentlichen gegenüberliegenden Richtung erstreckt wobei diese Muffe eine vordere Verlängerung aufweist, um diese aufblasbare Muffe (2) an der Röhre (1) anzuhaften.

2. Tracheakanüle nach Anspruch 1, wobei die Auf- und Abblasleitungen wenigstens eine biegsame Leitung (6) aufweisen.

3. Tracheakanüle nach Anspruch 1 oder 2, wobei die vordere Verlängerung einen Kragen (5) aufweist.

4. Tracheakanüle nach Anspruch 3, wobei der Kragen (5) entlang der Röhre bewegbar ist.

5. Tracheakanüle nach einem der Ansprüche 3 oder 4, wobei der Kragen (5) aufblasbar ist.

6. Tracheakanüle nach Anspruch 5, wobei der Kragen (5) mit der Muffe (2) in Verbindung ist.

7. Tracheakanüle nach einem der Ansprüche 1 bis 6, wobei die Muffe (2) nach Aufblasen eine derartige Größe aufweist, daß sie sich in den Grenzen des Trachealumens bis an den Larynx des Patienten erstreckt, derart daß sie die Röhre in der Trachea des Patienten einhält wenn die Tracheakanüle in die Trachea des Patienten eingeführt wird.

8. Eine Tracheotomiekanüle nach Anspruch 7, wobei der Kragen (5) auf dem ersten Teil der Röhre (1) angebracht ist, derart daß ein Luftausströmen durch die Tracheaöffnung vermieden wird, wenn die Tracheotomiekanüle in die Trachea des Patienten eingeführt wird.

## Revendications

1. Canule de trachéotomie destinée à être introduite dans la trachée d'un patient de façon à former un passage artificiel pour l'air à travers la gorge du patient, comprenant:
- un tube creux (1) présentant des parties d'extrémité proximale (3) et distale (4), et un coude entre lesdites parties d'extrémité de façon à former dans le tube des première et seconde portions dudit tube (1) qui comprennent respectivement lesdites parties d'extrémité proximale (3) et distale (4) dudit tube;
- un manchon gonflable (2) constitué d'un petit ballon;
- des conduits (6) pour gonfler et dégonfler ledit manchon (2);
caractérisée en ce que:
- ladite partie d'extrémité proximale (3) présente un rayon légèrement supérieur à celui de ladite première partie dudit tube (1), ladite seconde partie dudit tube s'étendant dans une première direction dans la trachée, après que ladite partie d'extrémité distale (4) ait été introduite dans la gorge du patient par une ouverture trachéale,
et en ce que
- ledit manchon (2), lorsqu'il est gonflé, s'étend à l'intérieur de la trachée du ou des patient(s) uniquement selon une deuxième direction sensiblement opposée à ladite première direction, ledit manchon comportant une extension antérieure destinée à fixer ledit manchon gonflable (2) sur ledit tube (1).

2. Canule de trachéotomie selon la revendication 1, dans laquelle lesdits conduits de gonflage et de dégonflage comprennent au moins un conduit flexible (6).

3. Canule de trachéotomie selon la revendication 1 ou 2, caractérisée en ce que ladite extension antérieure comprend un collier (5).

4. Canule de trachéotomie selon la revendication 3, dans laquelle ledit collier (5) est susceptible d'être déplacé le long dudit tube.

5. Canule de trachéotomie selon l'une quelconque des revendications 3 ou 4, dans laquelle ledit collier (5) est gonflable.

6. Canule de trachéotomie selon la revendication 5, dans laquelle ledit collier (5) communique avec ledit manchon (2).

7. Canule de trachéotomie selon l'une quelconque des revendications 1 à 6, dans laquelle ledit manchon (2) est d'une taille telle que, lorsqu'il est gonflé, il s'étende dans les limites du lumen de la trachée jusqu'au larynx dudit patient, de façon à retenir le tube dans la trachée du patient lorsque ladite canule de trachéotomie est introduite dans la trachée du patient.

8. Canule de trachéotomie selon la revendication 7, dans laquelle ledit collier (5) est disposé sur ladite première partie dudit tube (1), de façon à empêcher les fuites d'air à travers l'ouverture trachéale lorsque ladite canule de trachéotomie est introduite dans la trachée du patient.
